# EUROPEAN PATENT APPLICATION

(11) **EP 4 738 146 A1**
(43) Date of publication of application: **06.05.2026**
(21) Application number: 24210349.7
(22) Date of filing: 01.11.2024
(51) Int. Cl.: G06F 16/906, G06N 3/045, G16H 50/20

(54) **NEURAL NETWORK FOR VARIABLE NUMBER OF SIGNALS**

(71) Applicant: Koninklijke Philips N.V., 5656 AG Eindhoven (NL)
(72) Inventor: BRESCH, Erik, Eindhoven (NL); GROSSEKATHOEFER, Ulf, Eindhoven (NL)
(74) Representative: Philips Intellectual Property & Standards

(57) **Abstract**

Proposed concepts thus aim to provide schemes, solutions, concepts, designs, methods and systems pertaining to processing a variable number of clinical measurement signals. In particular, embodiments aim to provide a neural network for processing a variable number of clinical measurement signals by including a plurality of selectable input layers such that the neural network can adapt to the number of clinical measurement signals being input into it.

## Description

### FIELD OF THE INVENTION

This invention relates to the field of neural networks, and in particular to the field of neural networks for processing clinical measurement signals.

### BACKGROUND OF THE INVENTION

In a clinical environment, clinical measurement data, such as ECG data, typically consists of one or more lead signals. The number of clinical measurement signals present/available in any one situation, however, is variable. Furthermore, neural networks, typically have a fixed input tensor size, which makes it difficult to process multi-dimensional signals with varying dimensionality when deployed (e.g., 2-lead ECG vs 12-lead ECG).

Constructing a neural network for the maximum number of possible inputs and then zero-filling unused inputs is not a desirable solution, however, because the network then cannot intrinsically understand the difference between, for example, a zero signal because the input is unused, a zero signal because, for example, an ECG electrode fell off, and a zero signal because the subject has died.

Furthermore, the coefficients of neural networks can take up a sizable amount of storage space which is a valuable resource in embedded systems. Therefore, simultaneously deploying several networks (i.e., one for each possible input lead configuration) is a highly undesirable solution.

### SUMMARY OF THE INVENTION

The invention is defined by the claims.

According to examples in accordance with an aspect of the invention, there is provided a neural network for processing a variable number of clinical measurement signals.

The neural network comprises: a plurality of selectable input layers, each selectable input layer configured to, in use, receive an input clinical measurement signal, wherein each clinical measurement signal comprises respective clinical data of a same subject, and wherein each selectable input layer shares a first output tensor size; at least one selectable trunk having a first input tensor size, equal to the first output tensor size of the selectable input layers, and a second output tensor size; and at least one output layer configured to, in use, output at least one predicted feature of the one or more input clinical measurement signals; wherein the neural network is configured to, in use: select a number of the plurality of selectable input layers equal to the number of input clinical measurement signals.

Proposed concepts thus aim to provide schemes, solutions, concepts, designs, methods and systems pertaining to processing a variable number of clinical measurement signals. In particular, embodiments aim to provide a neural network for processing a variable number of clinical measurement signals by including a plurality of selectable input layers such that the neural network can adapt to the number of clinical measurement signals being input into it.

It is proposed that by providing a neural network structure which has multiple input layers which can be selected or not, the neural network can adapt to however many clinical measurement signals are input into it, and therefore be able to distinguish between situations where a signal is not present rather than the signal containing a value of zero (or just noise). This provides a highly efficient way of dealing with a variable number of clinical measurement signals, not requiring multiple entirely separate neural networks.

By ensuring that each input layer has the same output tensor size, which is equal to the input tensor sizes of each trunk present in the neural network, the neural network can effectively `mix and match' the different layers and trunks. Furthermore, by specifying that the number of input layers is selected to be equal to the number of input clinical measurement signals, the neural network remains as efficient as possible (in this respect) while also preventing situations where an input zero-signal is simply because, for example, an ECG lead is not being used, which would confuse the output of the neural network.

In addition, facilitating the provision of one or more selectable output layers allows each output layer to be specifically trained to output a specific predicted feature (or set of predicted features) of the clinical measurement signals.

Embodiments may be of particular use with ECG lead signals, as the number of ECG leads used to monitor a patient often varies depending on the context.

Ultimately, an improved neural network for processing a variable number of clinical measurement signals may be provided.

In some embodiments, the neural network may further comprise a plurality of selectable trunks, wherein each selectable trunk shares the first input tensor size and the second output tensor size; and wherein the neural network may be configured to, in use: select at least one of the plurality of selectable trunks based on available computational power of a processing arrangement running the neural network. This may provide a neural network structure which can adapt to the computational power of the processing arrangement which is running/executing said neural network, thus still allowing for a (potentially less accurate) result to be generated even in cases where the processing arrangement does not currently have the resources to run a more computationally-expensive trunk.

In some embodiments, the neural network may further comprise a plurality of streaming convolutional layers. These may allow the neural network to process continuous streams of data more efficiently.

In some embodiments, the plurality of streaming convolutional layers may be in at least one of the plurality of selectable trunks. This may be a particularly effective location for the streaming convolutional layers to facilitate the neural network running more efficiently.

In some embodiments, the neural network may further comprise, downstream of the plurality of streaming convolutional layers, at least one of: a long-short-term memory unit; and a general recursive unit. These units may thus process the direct or indirect outputs of the streaming convolutional layers, facilitating signal patterns to be connected across longer time frames.

In some embodiments, the neural network may further comprise a plurality of selectable output layers, each output layer being configured to, in use, output a different predicted feature of the one or more input clinical measurement signals. This may allow a user to select which predicted feature(s) of the input signals they are interested in obtaining, while also allowing each output layer to be specialized for outputting said predicted feature.

In some embodiments, the at least one predicted feature may comprise at least one of: wave delineation; detected beats; beat classification; an alert; ST segment length; rhythm class; and a modified version of at least one of the input clinical measurement signals. These may all be particularly useful features to predict from the input signals.

In some embodiments, weights of the neural network may be quantized and/or compressed. This may allow the neural network to be reduced in size and have faster inference times, which is especially beneficial for real-time applications.

In some embodiments, the one or more input clinical measurement signals may comprise at least one of: one or more input ECG lead signals; one or more input Pleth signals; and one or more input invasive blood pressure signals. These may all be signals for which the present invention is particularly beneficial for as the number of signals to be processed often varies from case to case.

In some embodiments, the one or more input clinical measurement signals may comprise one or more input ECG lead signals, and the neural network may comprise 12 selectable input layers. ECG monitoring typically involves up to 12 ECG lead signals, and thus this may be an efficient and effective number of selectable input layers to include in the neural network.

According to another aspect of the invention, there is provided a computer-implemented method for processing a variable number of clinical measurement signals. The method comprising: providing input data comprising one or more clinical measurement signals, wherein each clinical measurement signal comprises respective clinical data of a same subject, to a neural network, the neural network being trained to predict, from the input data, at least one feature of the input data; wherein the neural network comprises: a plurality of selectable input layers, wherein each selectable input layer shares a first output tensor size; at least one selectable trunk having a first input tensor size, equal to the first output tensor size of the selectable input layers, and a second input tensor size; and at least one output layer for outputting the at least one predicted feature of the input data; selecting a number of the plurality of selectable input layers equal to the number of clinical measurement signals.

According to another aspect of the invention, there is provided a computer-implemented method for training a neural network for processing a variable number of clinical measurement signals, the neural network comprising a plurality of selectable input layers, wherein each selectable input layer shares a first output tensor size; at least one selectable trunk having a first input tensor size, equal to the first output tensor size of the selectable input layers, and a second output tensor size; and at least one output layer for outputting at least one predicted feature of input data, wherein the method comprises: training the neural network on training data comprising a plurality of different numbers of clinical measurement signals.

In some embodiments, training the neural network may comprise training the neural network with augmentation. This may enhance the effectiveness of the training, resulting in a more reliable trained neural network.

In some embodiments, the augmentation may comprise at least one of: clinical measurement signal dropout; clinical measurement signal swap; gain charge; offset change; time-shift; and noise injection. These may all be effective augmentations for essentially enhancing the training data.

In some embodiments, the neural network may comprise n selectable input layers, and wherein the training data may comprise every permutation of the presence or absence of *n* clinical measurement signals. This may be a particularly effective way to train the neural network such that it is able to, in use, effectively process any permutation of up to *n* clinical measurement signals.

According to another aspect of the invention, there is provided a computer program comprising code means for implementing the method of any herein disclosed method when said program is run on a processing system.

Thus, there may be proposed concepts for a neural network processing a variable number of clinical measurement signals, and this may be done based on having the neural network include a plurality of selectable input layers and trunks.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of a neural network for processing a variable number of clinical measurement signals according to a proposed embodiment;
Fig. 2 is a block diagram of a neural network for processing a variable number of clinical measurement signals according to a proposed embodiment;
Fig. 3 is a diagram showing an example of selection of layers of a neural network according to a proposed embodiment;
Fig. 4 is a simplified flow diagram of a computer-implemented method for processing a variable number of clinical measurement signals according to a proposed embodiment;
Fig. 5 is a simplified flow diagram of a computer-implemented method for training a neural network for processing a variable number of clinical measurement signals according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to processing a variable number of clinical measurement signals. According to proposed concepts, a number of possible solutions may be implemented separately or jointly. That is, although these possible solutions may be described below separately, two or more of these possible solutions may be implemented in one combination or another.

Embodiments of the invention aim to provide a neural network for processing a variable number of clinical measurement signals. This can be achieved by including a plurality of selectable input layers such that the neural network can adapt to the number of clinical measurement signals being input into it.

In other words, it is proposed that by providing a neural network structure which has multiple input layers which can be selected or not, the neural network can adapt to however many clinical measurement signals are input into it, and therefore be able to distinguish between situations where a signal is not present rather than the signal containing a value of zero. This provides a highly efficient way of dealing with a variable number of clinical measurement signals, not requiring multiple entirely separate neural networks.

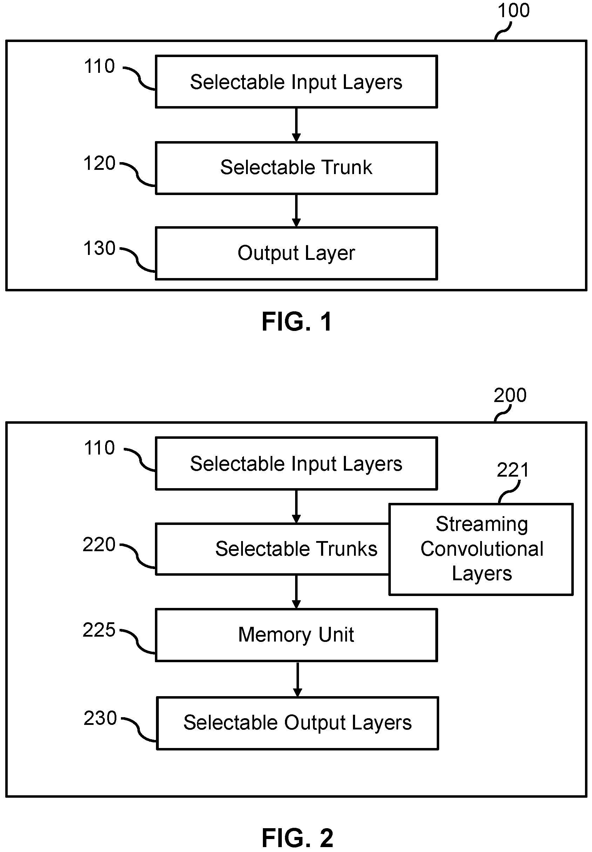

Referring now to Fig. 1, there is depicted a simplified block diagram of a neural network 100 for processing a variable number of clinical measurement signals according to a proposed embodiment.

The neural network 100 comprises a plurality of selectable input layers 110, each selectable input layer configured to, in use, receive an input clinical measurement signal, wherein each clinical measurement signal comprises respective clinical data of a same subject, and wherein each selectable input layer shares a first output tensor size. In other words, each selectable input layer is configured to, in use, receive a single clinical measurement signal (e.g., an ECG lead signal). Each clinical measurement signal (e.g., an ECG lead signal) comprises respective clinical data (e.g., ECG data) of the same subject. For example, if there were three input ECG lead signals, each would include ECG data of the same subject. Furthermore, each input layer has the same first output tensor size (i.e., such that all the input layers are matching with regards to their output tensor size). Tensor size can also be understood as tensor shape, or the number of dimensions of the tensor.

As the skilled person would understand, input layers are neural network layers which are close to the input of the network and typically perform the initial processing or feature extraction of the input data. By having multiple selectable input layers, the network can have several different input processing pathways.

In some embodiments, the one or more input clinical measurement signals can comprise at least one of: one or more input ECG lead signals; one or more input Pleth signals; and one or more input invasive blood pressure signals. These are all signals for which the present invention is particularly beneficial for as the number of signals to be processed often varies from case to case. For example, the neural network will be specifically trained to process one of the different types of clinical measurement signals provided above and will not generally `mix and match'. For example, in an embodiment, the neural network can be specifically trained to process ECG lead signals, and thus the input clinical measurement signals will consist of one or more input ECG lead signals. In other embodiments, as the skilled person would understand, any other suitable types of clinical measurement signals can be used.

As the skilled person would know, a Pleth signal can also be referred to as a photoplethysmogram (PPG) signal which is a waveform that measures changes in blood volume in the microvascular bed of tissue, typically obtained using a pulse oximeter, and reflects the cardiovascular pulse wave. As the skilled person would know, an invasive blood pressure signal is a continuous waveform that directly measures arterial blood pressure through a catheter inserted into a blood vessel.

In fact, in this embodiment, the one or more input clinical measurement signals specifically comprise one or more input ECG lead signals. The neural network also specifically comprises twelve selectable input layers 110. ECG monitoring typically involves up to 12 ECG lead signals, and thus this is an efficient and effective number of selectable input layers 110 to include in the neural network 100. It is noted that for input ECG lead signals (which are physical electrical input signals), they are sampled at a rate between 100Hz and 500Hz.

The neural network 100 also comprises at least one selectable trunk 120 having a first input tensor size, equal to the first output tensor size of the selectable input layers, and a second output tensor size.

As the skilled person would understand, a trunk of a neural network is the main body of said neural network which performs complex processing and feature extraction. They typically contain multiple layers and form the core of the network architecture. Typically, neural networks only include a single trunk, however, in the present invention, multiple trunks can be present which are selectable (i.e., it can be chosen which trunk to use in the processing pathway).

The neural network 100 also comprises at least one output layer 130 which is configured to, in use, output at least one predicted feature of the one or more input clinical measurement signals. As the skilled person would understand, output layers are specialized components of neural network architecture which are designed to process and format the network's output for a particular task/objective. It is the final layer of the neural network following the trunk(s), and it can transform the high-level representations of the trunk network into the desired output format for the task for which it is trained (i.e., outputting the predicted feature).

It should be noted that it is beneficial that each of the at least one output layers has a third (matching) input tensor size which is equal to the second output tensor size of the trunks.

In this embodiment, the at least one predicted feature comprises at least one of: wave delineation; detected beats; beat classification; an alert; ST segment length; rhythm class; and a modified version of at least one of the input clinical measurement signals. These are all particularly useful features to predict from the input signals. Wave delineation refers to the identification of key points in the signal (e.g., P, QRS, T waves in an ECG lead signal). Detected beats refers to the identification of individual heart beats within the signal. Beat classification refers to the classification of identified/detected beats (e.g., normal or abnormal). An alert refers to a notification/indication that there is something abnormal / clinically concerning with the subject as indicated in the input signals. An ST segment length refers to the duration of an ST segment, which is a concept known to the skilled person (the section between the end of an S wave and the start of a T wave). Rhythm class refers to an overall classification of the heart's rhythm (e.g., sinus rhythm, atrial fibrillation, bradycardia, tachycardia, etc.). A modified version of a clinical measurement signal refers to a processed or filtered version of one of the input clinical measurement signals (e.g., to enhance certain features, reduce noise, etc. for subsequent external analysis or display to a user).

Of course, in other embodiments, as the skilled person would appreciate, any suitable/useful features of the input data can be predicted.

It is noted that output signals of the at least one output layer 130 can be binary, one-hot encoded, and/or probability signals.

In use, the neural network 100 is configured to: select a number of the plurality of selectable input layers 110 equal to the number of input clinical measurement signals. The skilled person would understand therefore that the maximum number of input clinical measurement signals is dictated by the number of selectable input layers 110 present in the neural network 100.

As noted, in some embodiments, the neural network 100 can comprise a plurality of selectable trunks (i.e., more than just one), wherein each selectable trunk shares a first input tensor size, equal to the first output tensor size of the selectable input layers, and a second output tensor size. In other words, each of the multiple trunks can have the same input tensor size (i.e., are matching in this regard), and furthermore, this input tensor size is equal to the first output tensor size of the input layers. Each of the multiple trunks can also have the same output tensor size (i.e., are matching in this regard). By providing multiple selectable trunks, each can have different requirements for the computational power required to run them - typically, the more computational power required, the better (e.g., more accurate, faster, etc.) the neural network. In these embodiments (i.e., including multiple trunks), the neural network can thus be configured to select at least one of the plurality of selectable trunks based on available computational power of a processing arrangement running the neural network. It should be noted that in most embodiments, only one of the plurality of selectable trunks will be selected, however, in some embodiments, more than one can be selected, e.g., for parallel processing. It should also be noted that in some embodiments, the selection of at least one of the plurality of trunks can also be further based on the number of input clinical measurement signals.

The available computational power of the processing arrangement/system running/executing the neural network (for inference) can be understood as the capacity of the processing arrangement (e.g., one or more CPUs, GPUs, TPUs) to handle tasks at a given time, referring to factors such as processing speed, parallelism capabilities, memory availability, current workload, thermal limits, etc. The skilled person would understand the many ways in which the computational power of the processing arrangement could be quantified and assessed but what is important to this invention is that in some way the available computational power of the processing arrangement is taken into account when selecting which of the multiple trunks to use (in embodiments where the neural network includes multiple trunks). For instance, one trunk may be more resource intensive than the other trunk and so only be selected if there is sufficient computational power available to run it.

In other words, the available computational resources for the execution of the neural network may change during runtime in a computational environment, for example, with multi-tasking support several applications simultaneously. It is therefore desirable to be able to scale up or down the computational effort/resources to run the neural network. Of course, a lower-effort computational-effort neural network will typically have worse performance.

In another aspect of the invention, there can thus be provided a system comprising a processing arrangement and the neural network 100 (in some embodiments, including a plurality of selectable trunks), wherein the processing arrangement is configured to run/execute the neural network 100 (for inference).

It should also be noted that in some embodiments, weights of the neural network 100 can be quantized and/or compressed. This may allow the neural network to be reduced in size and have faster inference times, which is especially beneficial for real-time applications. As the skilled person would understand, quantization of weights reduces the precision of the neural network's weights (e.g., from 32-bit floats to 8-bit integers), which decreases the model's size and computation requirements while maintaining acceptable performance. Compression of weights reduces the storage and computational load of a neural network by using techniques such as pruning, low-rank factorization, or entropy coding to remove redundancy and represent weights more efficiently.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

In summary, the structure of the neural network of the present invention facilitates distinguishing between the following potential causes of the absence of an input signal. For example, for an ECG lead, the absence of a signal may be due to:
The electrode/lead being defective (e.g., dry or fell of): in this case, a signal would be delivered but it would merely be noise - it is desired that a signal being absent due to this cause is ignored (e.g., no alarm is raised) which is likely to be the case as long as other lead signals are present and the network is robust, e.g., through training augmentation;
The electrode/lead is present and the signal is truly zero, i.e., this implies that a patient has died and there is no heart activity - a signal being absent due to this cause must not be ignored and an alarm should be raised; and
The electrode/lead is knowingly not present in the ECG setup (e.g., signal may be represented as a stream of 'zero' samples) - a signal being absent due to this cause should be ignored and no alarm should be raised.

A composite network architecture is thus provided which can distinguish between the above scenarios and efficiently deal with a variable number of clinical measurement signals. In an example, the architecture can include: several selectable (shallow) input layers to support any possibly occurring number of input signals. Critically, these input layers should have the same common output tensor size which allows them all to connect to the same trunk neural network; one or more selectable trunk neural networks with common input and output tensor size (by providing multiple trunks, scaling up or down the computational effort needed to run the neural network can be facilitated); and at least one task-specific output layer.

For example, during training, all possible system configurations should be jointly trained on data from all possible input signal configurations, i.e., by means of random configuration choice for each training step. A neural network structured and trained according to the above would thus be able to distinguish between the causes of `zero input signal' listed above (and most importantly between causes 2 and 3).

The present invention would thus be especially beneficial for use, for example, in smart alarms in an ICU, real-time fetal (and maternal) monitoring and diagnostics, ECG analysis on smart devices such as smart watches, or any other signal monitoring applications, such as EEG analysis (e.g., for sleep staging), or PPG analysis.

Referring now to Fig. 2, there is depicted a flow diagram of a neural network 200 for processing a variable number of clinical measurement signals according to a proposed embodiment. The selectable input layer 110 are substantially the same as those described in relation to neural network 100 of Fig. 1.

In this embodiment, the neural network 200 comprises a plurality of selectable trunks 220 (as discussed as a possibility above). Furthermore, at least one of the plurality of selectable trunks 220 comprise a plurality of streaming convolutional layers 221. This can thus allow the neural network to more efficiently process continuous streams of data. In other embodiments, as would be understood by the skilled person, other layers (such as the input layers 110) can, alternatively or in addition, comprise a plurality of streaming convolutional layers. As the skilled person would know, streaming convolutional layers are adaptations of traditional convolutional layers designed to process continuous streams of data efficiently, without needing to store or process the entire input sequence/signal at once. In other words, the streaming convolutional layers are configured to update internal states of the model with new data (e.g., while continuously running).

For example, neural networks often struggle to be effectively implemented on resource-constrained embedded devices, such as wearables or medical patient monitors. These devices typically have limited computational power and energy efficiency which, for example, would hamper real-time processing of clinical measurement signals (such as ECG lead signals) using deep neural networks. By including streaming convolutional layers in the neural network, which allow internal states to be updated with new data, clinical measurement signals can be processed sample-by-sample as they arrive which ensures efficient processing and analysis of real-time clinical measurement signals without compromising accuracy. The implementation of streaming convolutional layers also allows the implementation of filtering components which process incoming signals one sample at a time, reducing the need to implement costly first-in, first-out (FIFO) elements, therefore also eliminating the need for external error-prone preprocessing of signals outside the deep neural network.

In this embodiment, the neural network 200 also comprises a memory unit 225 (downstream of the plurality of streaming convolutional layers 221). The memory unit 225 comprises at least one of: a long-short-term memory (LSTM) unit; and a general recursive unit. These units process the direct or indirect outputs of the streaming convolutional layers 221, facilitating signal patterns to be connected across longer time frames.

As would be known to the skilled person, an LSTM unit is a type of recurrent neural network unit designed to capture long-term dependencies in sequences by using gating mechanisms to regulate the flow of information. Likewise, a general recursive unit processes hierarchical or structured data by recursively applying the same set of weights over a structured input, enabling the model to handle tree-like or recursive patterns.

In this embodiment, the neural network 200 also comprises a plurality of selectable output layers 230, each output layer being configured to, in use, output a different predicted feature of the one or more input clinical measurement signals. In this way, for example, a user can select which predicted feature of (one or more of) the input signals they are interested in obtaining, while also allowing each output layer to be specialized for outputting said predicted feature.

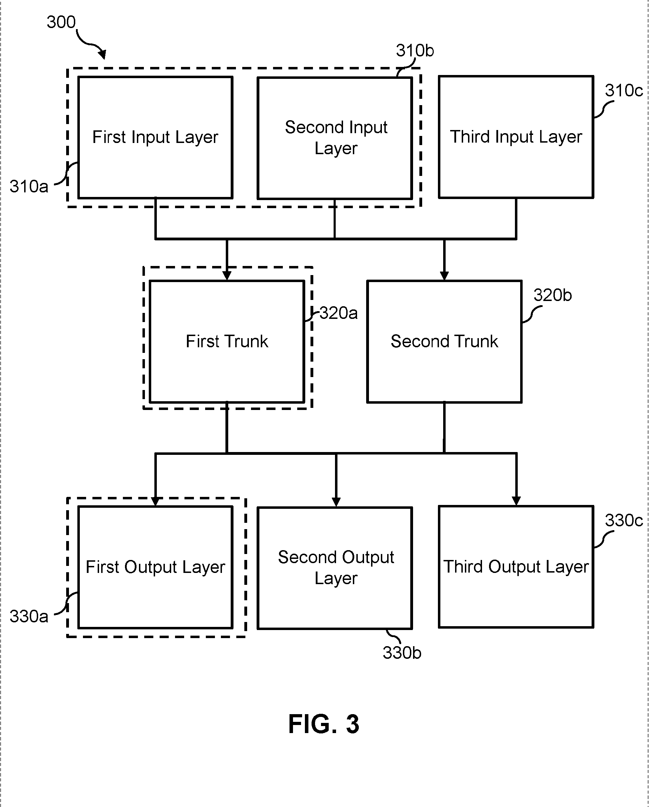

Referring now to Fig. 3, there is depicted a diagram showing an example of the selection of layers of a neural network 300 according to a proposed embodiment.

For example, this neural network comprises: three selectable input layers, 310a-3 10c; two selectable trunks, 320a and 320b; and three selectable output layers, 330a-330c. In this example, two clinical measurement signals are input into the neural network 300 - accordingly, two of the input layers are selected, 310a and 310b (indicated by the dashed box), such that the number of input layers equals the number of input clinical measurement signals. For example, if another clinical measurement signal were added to the input data, the third input layer 310c could also be selected.

In this example, trunk 320a has a low computational cost but has lower performance whereas trunk 320b has a higher computational cost and higher performance. In this example, the processing arrangement running the neural network does not have enough available computational power to run the more intensive trunk 320b, and thus the simpler trunk 320a is selected (indicated by the dashed box).

Each of the three selectable output layers 330a-330c have been trained for outputting a different predicted feature (e.g., layer 330a has been trained to output predicted wave delineation, layer 330b has been trained to output predicted beat detection, and layer 330c has been trained to output predicted beat classification). In this example, the user is only interested in wave delineation and thus only layer 330a is selected, though if they wish to obtain more predicted features, they could select more of the output layers.

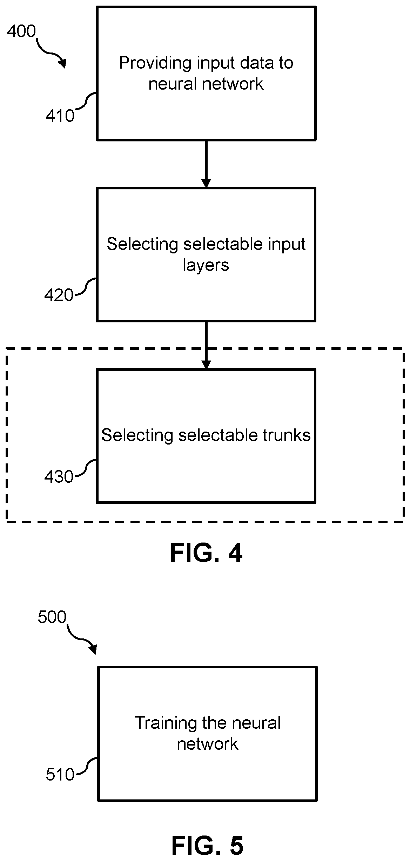

Referring now to Fig. 4, there is depicted a simplified flow diagram of a computer-implemented method 400 for processing a variable number of clinical measurement signals according to a proposed embodiment.

The method comprises step 410 of providing input data comprising one or more clinical measurement signals, wherein each clinical measurement signal comprises respective clinical data of a same subject, to a neural network (e.g., neural network 100). In this embodiment, the neural network being trained to predict, from the input data, at least one feature of the input data; wherein the neural network comprises: a plurality of selectable input layers, wherein each selectable input layer shares a first output tensor size; at least one selectable trunk having a first input tensor size, equal to the first output tensor size of the selectable input layers, and a second input tensor size; and at least one output layer for outputting the at least one predicted feature of the input data.

Step 420 comprises selecting a number of the plurality of selectable input layers equal to the number of clinical measurement signals. In embodiments where the neural network comprises a plurality of selectable trunks, the method 400 can also comprise step 430 comprising selecting at least one of the plurality of selectable trunks based on available computational power of a processing arrangement running the neural network. It is noted that the neural network cannot start substantially processing the input data provided to it in step 410 until step 420 (and 430 if multiple trunks are present) have been completed (i.e., until the layers the neural network will use to process the input data have been selected).

Accordingly, in embodiments where the neural network comprises a plurality of output layers, the method 400 can, of course, also comprise a step (not shown) of selecting at least one of the multiple output layers. This step can be performed automatically (e.g., based on some criteria) or it can be performed by a user (i.e., manual selection).

Referring now to Fig. 5, there is depicted a computer-implemented method 500 for training a neural network (e.g., neural network 100) for processing a variable number of clinical measurement signals. In this embodiment, the neural network comprises a plurality of selectable input layers, wherein each selectable input layer shares a first output tensor size, at least one selectable trunk having a first input tensor size, equal to the first output tensor size of the selectable input layers, and a second output tensor size; and at least one output layer for outputting at least one predicted feature of input data.

The method 500 comprises step 510 of training the neural network on training data comprising a plurality of different numbers of clinical measurement signals. In other words, the training data is clustered into individual chunks/sets/groups, wherein the individual chunks/sets/groups comprise different numbers of clinical measurement signals compared to one another. In yet other words, the training data is organized into subsets where the subsets contain varying numbers of clinical measurement signals, for example, one subset may comprise two clinical measurement signals (of a same subject) and another subset may comprise four clinical measurement signals (of a same subject). In this way, the model can learn to generalize across cases where different numbers of signals are available.

In this embodiment, training the neural network comprises training the neural network with augmentation. This enhances the effectiveness of the training (by artificially increasing the diversity of training data), resulting in a more reliable trained neural network (which is able to more effectively generalize), though as the skilled person would appreciate, not essential to the working of the method 500. In this embodiment, the augmentation comprises at least one of: clinical measurement signal dropout; clinical measurement signal swap; gain charge; offset change; time-shift; and noise injection. These are all effective augmentations for essentially enhancing the training data, though as the skilled person would appreciate, in other embodiments, the augmentation can comprise any other suitable form of augmentation.

Clinical measurement signal dropout can be understood as removing certain clinical measurement signals during training to simulate missing data. Clinical measurement signal swap can be understood as exchanging one clinical signal with another. Gain change can be understood as altering the amplitude of a signal by scaling its values. Offset change can be understood as shifting the baseline of a signal by adding a constant value. Time-shift can be understood as shifting the signal forward or backward in time. Noise injection can be understood as adding noise to a signal.

In some embodiments, the neural network can specifically comprise n selectable input layers, and wherein the training data comprises every permutation of the presence or absence of *n* clinical measurement signals. This is a particularly effective way to train the neural network such that it is able to, in use, effectively process any permutation (of the presence or absence) of up to *n* clinical measurement signals.

In other words, the training of a neural network according to the present disclosure can be optimized by augmenting the training with additional targets, injecting expert knowledge into the model. For example, in an application where the network is trained to assign single instances of beats to a beat class, an additional target could be provided by training the network to also detect different wave segments within the signal (e.g., an ECG). While this additional target is not directly related to beat classification, such an approach would inject expert knowledge about the signals and their morphology and semantics into the training process, potentially allowing the deep neural network to leverage and cross-connect different tasks to gain better understanding of the underlying processes generating the signals. For example, to implement this, additional targets can be injected in the training process as auxiliary targets.

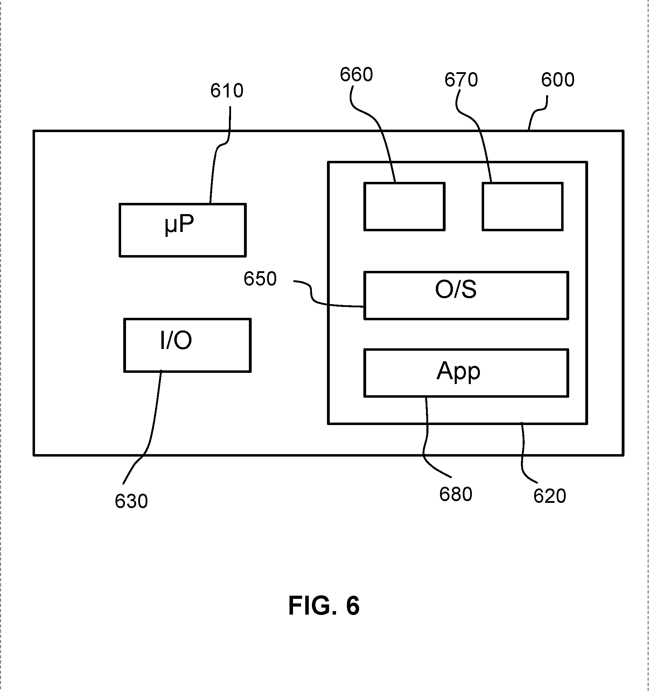

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The neural networks of Figs. 1 and 2, and the methods of Figs. 4 and 5 be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A neural network (100) for processing a variable number of clinical measurement signals, the neural network comprising:
a plurality of selectable input layers (110), each selectable input layer configured to, in use, receive an input clinical measurement signal, wherein each clinical measurement signal comprises respective clinical data of a same subject, and wherein each selectable input layer shares a first output tensor size;
at least one selectable trunk (120) having a first input tensor size, equal to the first output tensor size of the selectable input layers, and a second output tensor size; and
at least one output layer (130) configured to, in use, output at least one predicted feature of the one or more input clinical measurement signals;
wherein the neural network is configured to, in use:
select a number of the plurality of selectable input layers equal to the number of input clinical measurement signals.

2. The neural network of claim 1, further comprising a plurality of selectable trunks (220), wherein each selectable trunk shares the first input tensor size and the second output tensor size; and wherein the neural network is configured to, in use: select at least one of the plurality of selectable trunks based on available computational power of a processing arrangement running the neural network.

3. The neural network of claim 1 or 2, further comprising a plurality of streaming convolutional layers (221).

4. The neural network of claim 3, wherein the plurality of streaming convolutional layers are in at least one of the plurality of selectable trunks.

5. The neural network of claim 3 or 4, further comprising, downstream of the plurality of streaming convolutional layers, at least one of: a long-short-term memory unit (225); and a general recursive unit (225).

6. The neural network of any of claims 1 to 5, further comprising a plurality of selectable output layers (230), each output layer configured to, in use, output a different predicted feature of the one or more input clinical measurement signals.

7. The neural network of any of claims 1 to 6, wherein the at least one predicted feature comprises at least one of: wave delineation; detected beats; beat classification; an alert; ST segment length; rhythm class; and a modified version of at least one of the input clinical measurement signals.

8. The neural network of any of claims 1 to 7, wherein weights of the neural network are quantized and/or compressed.

9. The neural network of any of claims 1 to 8, wherein the one or more input clinical measurement signals comprise at least one of: one or more input ECG lead signals; one or more input Pleth signals: and one or more input invasive blood pressure signals.

10. A computer-implemented method (400) for processing a variable number of clinical measurement signals, the method comprising:
providing input data (410) comprising one or more clinical measurement signals, wherein each clinical measurement signal comprises respective clinical data of a same subject, to a neural network, the neural network being trained to predict, from the input data, at least one feature of the input data;
wherein the neural network comprises:
a plurality of selectable input layers, wherein each selectable input layer shares a first output tensor size;
at least one selectable trunk having a first input tensor size, equal to the first output tensor size of the selectable input layers, and a second output tensor size; and
at least one output layer for outputting the at least one predicted feature of the input data;
selecting a number of the plurality of selectable input layers (420) equal to the number of clinical measurement signals.

11. A computer-implemented method (500) for training a neural network for processing a variable number of clinical measurement signals, the neural network comprising a plurality of selectable input layers, wherein each selectable input layer shares a first output tensor size; at least one selectable trunk having a first input tensor size, equal to the first output tensor size of the selectable input layers, and a second output tensor size; and at least one output layer for outputting at least one predicted feature of input data, wherein the method comprises:
training the neural network (510) on training data comprising a plurality of different numbers of clinical measurement signals

12. The computer-implemented method of claim 11, wherein training the neural network comprises training the neural network with augmentation.

13. The computer-implemented method of claim 12, wherein the augmentation comprises at least one of: clinical measurement signal dropout; clinical measurement signal swap; gain change; offset change; time-shift; and noise injection.

14. The computer-implemented method of any of claims 11 to 13, the neural network comprises n selectable input layers, and wherein the training data comprises every permutation of the presence or absence of n clinical measurement signals.

15. A computer program comprising code means for implementing the method of any of claims 10 to 14 when said program is run on a processing system.
